# EUROPEAN PATENT APPLICATION

(11) **EP 1 958 599 A1**
(43) Date of publication of application: **20.08.2008**
(21) Application number: 07102235.4
(22) Date of filing: 13.02.2007
(51) Int. Cl.: A61F 6/14, A61F 6/18

(54) **An IUS inserter and a kit**

(71) Applicant: Familplan Consulting Ltd., 01531 Vantaa (FI)
(72) Inventor: Luukkainen, Tapani, 02160 Espoo (FI)
(74) Representative: Maskula, Silla Marjatta

(57) **Abstract**

The invention relates to an IUS inserter (1) comprising a tube (2) having a first end opening (2') and a second end opening (2''). A hollow space extends from the first end opening to the second end opening. A flange (3) is arranged around the outer surface of the tube, and a plunger (5) is arranged partly inside the tube through the second end opening and being movable back-and-forth along the length axis of the tube. The flange is stationary arranged to a first distance from the first end opening of the tube. The invention relates also to a kit comprising the inserter.

## Description

The invention relates to an IUS inserter, a kit and to an insertion method of an IUS as defined in the preambles of the appended claims.

### BACKGROUND OF THE INVENTION

Insertion of intrauterine systems (IUS) is a procedure that requires care and practice. For example, IUS must be inserted deep enough into the uterine cavity so that the IUS is not inadvertently expelled during the use, which might lead to an unwanted pregnancy. Normally, insertion of an IUS is started by measuring the depth of the uterine cavity by using a thin metal sound. The sound has a centimetre scale marked on it, and by pushing it to the end of the uterine cavity the depth of the uterine cavity is determined. After that the length of the IUS inserter is adjusted to correspond to the determined depth of the uterine cavity, and the IUS is placed into the inserter. With the aid of the inserter the IUS is brought to the fundal part of the uterus and released.

However, there are some drawbacks in the present procedure. The end of the used metal sound is relatively sharp. There is a risk that the sound is accidentally pushed too far into the uterine cavity, in which case the uterus may become perforated. Consequently, the depth of the uterus may also become wrongly determined and the IUS might become inserted into the uterine wall, even into the abdominal cavity. Correctly inserted IUS may also seek by itself to the perforation in the uterine wall.

All these risks require that the medical practitioners must be carefully and extensively educated to insert the IUS in order to avoid the above-mentioned drawbacks. Preferably, only gynaecologists having extensive training and experience would insert IUSs. The required education of the medical practitioners can be relatively easily arranged in the developed countries, where also specialist practitioners are widely available. However, the situation is completely different in the developing countries. At the same time, there is a great need for safe and reversible contraceptive methods providing long-time protection against unwanted/recurring pregnancies in the developing countries. The IUS's could offer a perfect solution as a long-term contraceptive, but their demanding insertion hinders them becoming generally available. Furthermore, in the developing countries it is usually midwifes or relatively low educated general medical practitioners that provide the contraceptive methods. Therefore there is great need to simplify the insertion procedure of the IUSs, preferably drug releasing intrauterine systems.

### OBJECT OF THE INVENTION

The object of the invention is to minimise or even eliminate the problems existing in the prior art.

An object of the present invention is to provide a simple and easy-to-use inserter for an IUS.

Another object of the invention is to provide a kit comprising an inserter and an IUS that would be cheap and simple to manufacture and use.

### SUMMARY OF THE INVENTION

In order to achieve the above-mentioned objects the present invention is characterised in what is defined in the characterising parts of the independent claims presented hereafter.

Typical IUS inserter according to the present invention comprises
- a tube having a first end opening and a second end opening and defining a hollow space extending from the first end opening to the second end opening,
- a flange arranged around the outer surface of the tube,
- a plunger arranged partly inside the tube through the second end opening and being movable back-and-forth along the length axis of the tube.
   The flange is stationary fixed to a first distance from the first end opening of the tube.
   Typical kit according to the present invention comprises
- an inserter according to any of the claims 1 to 5, and
- an intrauterine system comprising
   - a T-shaped frame consisting of an elongate member having at its first end a transverse member comprising a first wing and a second wing, the first ends of said wings being attached to said elongate member, and the first and second wings being symmetrically bent towards a second end of said elongate member so that an angle α is formed between said elongate member and said first or second wing; and the second ends of the wings are symmetrically bent towards the elongate member by an angle β that is 40°-140°, and
   - a therapeutic system.

Typical method according to the present invention for inserting an IUS into uterine cavity comprising following steps:
- loading an IUS to an IUS inserter tube through a first end opening of the tube,
- inserting the IUS inserter loaded with the IUS into the uterine cavity through vagina and cervical canal,
- releasing the IUS into the uterine cavity by using the inserter,
- using an inserter comprising a flange, which is stationary arranged to a first distance from the first end opening of the tube without any measurement of the depth of the uterine cavity before or during the insertion procedure.

### DETAILED DESCRIPTION OF THE INVENTION.

Now it has been surprisingly found out that in most cases the insertion of an IUS can be made simply and safely in the middle part of the uterine cavity without risk of undesired expulsion by using the inserter according to the present invention. According to the present invention, an inserter having a stationary flange can be used, whereby the fixation point of the flange is selected appropriately so that it reaches the middle part of the uterine cavity for most of the insertion objects, but not longer. The flange prevents the possibility that the inserter is pushed too deep into the fundal part of the uterus and minimises the risk of perforation of the uterus wall. The use of the inserter makes also the determination of the depth of the uterine cavity superfluous and thus eliminates the perforations made by the sound. Because the inserter makes it practically impossible to incur uterus perforation during the insertion, also persons with lower experience and/or lower medical education levels, such as midwifes and general practitioners, can safely insert IUS by using the inserter according to the present invention. The use of this invention results that insertions leave the IUS in the correct place and position in the uterine cavity. The use of the inserter is also cheap, as no specialised equipment, such as sounds, are require, which makes the inserter very suitable to be used in the developing countries.

According to the invention the flange is stationary fixed around the inserter tube at a point where the distance between the first end of the inserter tube and the flange is long enough so that the first end of the inserter is located at the middle part of the uterine cavity during the insertion. According to one embodiment of the invention this first distance from the first end opening of the tube to the flange is normally 4.5 - 6.5 cm, preferably 5 - 6 cm, more preferably 5.3 - 5.6 cm. It has been noticed that these values are suitable for insertion of the IUS deep enough into the uterine cavity without the risk of uterine wall perforation.

Preferably the tube of the inserter according to the invention is malleable and it is made of plastic. All plastics that are suitable for medical applications can be used. As the inserter is not hold in bodily cavity for extensive periods of time, it does not need to fulfil any strict requirements. It is sterile and preferably insoluble. Malleable means that the tube is not rigid, and it can be bended without extensive force. The tube adjusts easily during the insertion after the position of the uterus and the cervical canal. The edges of the first end opening of the inserter tube are preferably smooth and rounded, so that the possibility for accidental injury to the tissues during the IUS insertion is minimised.

The outer diameter of the inserter tube is typically 5.5 mm or less, more typically 2.5 - 5.2 mm, preferably 3 - 5 mm. The outer diameter of the tube can be selected according to the size of the IUS that is to be inserted. As the inserter according to the present invention is simple and easy to manufacture, it is feasible to manufacture inserters with different inner diameters, suitable for different IUS.

Normally the thickness of the tube wall is 0.05 - 0.3 mm, typically 0.1 - 0.2 mm.

The stationary flange is arranged to a second distance from the second end opening of the inserter tube, the second distance being typically 20 - 30 cm, preferably 22 - 27 cm. The second distance is selected so that the use of the inserter is easy and uncomplicated.

The flange may be arranged stationary around the outer surface of the inserter tube by using suitable fixation aid, such as glue. The fixation aid is selected so that it is acceptable for medicinal purposes, does not cause any harmful side effects and guarantees a strong long-term fixation. The flange and the tube may also be manufactured as one piece, for example by injection moulding. It is also possible to fix the flange stationary by forming to the fixation point of the tube and the flange suitable mechanical fasteners, such as threads, claws or barbs, which can be attached to each other.

The size of the flange is selected so that it cannot enter into the cervical canal. The width of the flange in its diametrical direction is typically 2 - 4 mm, preferably 2.5 - 3.5 mm, and the length of the flange in axial direction of the tube is 2 - 5 mm. The flange is also preferably rounded and smooth without any sharp edges or the like. The width of the flange is usually measured from the connection point of the outer surface of the tube and flange to the outer edge of the flange. Sometimes the width of the flange in its diametrical direction is typically 7 - 9 mm, preferably 6.5 - 8.5 mm with a round hole for the tube in the centre.

As the inserter is cheap to manufacture it is preferably disposable, i.e. intended to be used only once. Therefore the inserter material can be chosen so that it does not have to withstand sterilisation. Disposable inserters are preferable in developing countries where the complete and adequate sterilisation of medical instruments like the sound or inserter is often difficult to achieve. Poorly done sterilisation increases the risk for infections, e.g. HIV. These problems are solved by using the inserter according the present invention.

The plunger is arranged to the second end of the inserter tube. Usually there is a holding means, such as ring or handle, arranged in the first end of the plunger and the plunger body shows a mark, which indicates the suitable insertion depth for the plunger. By using the holding means the plunger is movable back-and-forth inside the tube. During the IUS insertion the plunger is first moved to inwards towards the cervical canal up to the mark on the plunger body. Then the tube around the plunger is moved in the cervical canal to the point where the flange prevents the movement of the tube. Then the tube is moved backwards away from the uterus by pushing the tube on the stationary plunger until the handle of the plunger and the IUS is inserted from the first end of the tube and released out of the inserter tube into the correct position in the uterine cavity. The holding means function also as stopper for the movement during the IUS insertion, the holding means preventing the pushing of the IUS too far in.

Preferably the inserter according to the present invention is used for insertion of a T-shaped IUS, where the first and second wings are symmetrically bent towards a second end of the elongate member. The angle α formed between the elongate member and the first or second wings is typically 10°-60°, preferably 20° - 50°, more preferably 30° - 50°. According to one embodiment of the invention the second ends of the IUS wings are symmetrically bent towards the elongate member by an angle β that is usually 40°-140°, preferably 60°-130°, more preferably 90°-120°.

According to one preferred embodiment of the invention the elongate member of the IUS further has a rounded knob at its first end wherein the first and second wings are attached to the knob.

The therapeutic system of the IUS can be a reservoir for a therapeutically active agent, for example a frame made of an elastomer and comprising a therapeutically active agent. Such systems comprising therapeutically active agents have been disclosed in the prior art and are well known for the person skilled in the art. The therapeutic system may be, for example, also a metal wire, such as a copper wire. Preferably, however, the therapeutic system is a steroid reservoir, which releases a suitable steroid at suitable, well-controlled rate. Preferably the therapeutic system is a drug containing cylinder coated with a release-rate controlling membrane. The therapeutic system is preferably positioned on the elongate member of the IUS frame, but it may also be positioned in one or both of the wings.

The wings of the IUS frame may be left outside the inserter during the insertion.

### DRAWINGS

The invention is described below in greater detail by the following, non-limiting drawing.

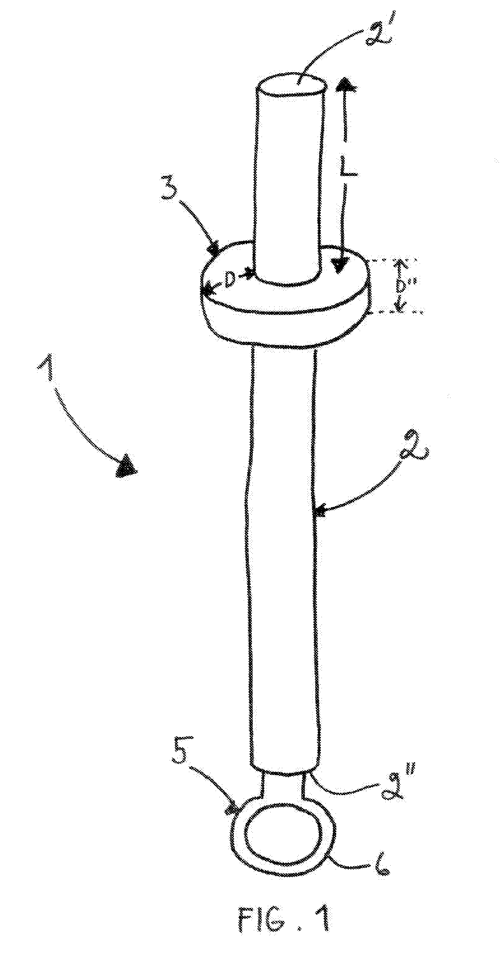

Figure 1 illustrates an IUS inserter according to one embodiment of the present invention.

Figure 1 shows an IUS inserter according to one embodiment of the invention. The inserter 1 comprises a hollow tube 2 having a first end opening 2' and a second end opening 2". During the insertion procedure the IUS is arranged inside the tube 2 through the first end opening.

At a distance L from the first end opening 2' is arranged a flange 3 that is stationary in relation to the tube 3. In other words the flange 3 cannot be moved along the outer surface of the tube 2. The flange 3 prevents the insertion of the tube too deep into the uterine cavity. The length L is chosen so that the first end 2' of the tube 2 reaches the middle part of the uterine cavity. The width D of the flange 3 is typically measured from connection point between the outer surface of the tube 2 and the outer edge 3' of the flange. The length D' of the flange is measured in the axial direction of the tube 2.

In the second end opening 2" of the tube 2 is arranged a plunger 5 having a ring handle 6 in its second end. In Figure 1 the plunger 5 is shown in second extreme position where it is inside the tube 2. Usually the plunger is in this position when the IUS is to be released into the uterine cavity. During typical IUS insertion procedure the plunger is usually held immovable in the uterine cavity and the tube is carefully pulled away to the direction of the second end of the tube. Then the IUS is released out of the tube to a proper place in the uterine cavity and the plunger is carefully pulled out towards the second end of the tube. Finally the tube is completely removed out of the uterine cavity.

It is to be understood that in Figure 1 the dimensions of the different parts of the inserter are not true to life, nor in proportion to each other.

It will be appreciated that the present invention can be incorporated in the form of a variety of embodiments, only a few of which are disclosed herein. It will be apparent for the specialist in the field that other embodiments exist and do not depart from the spirit of the invention. Thus, the described embodiments are illustrative and should not be construed as restrictive.

## Claims

1. An IUS inserter comprising
- a tube having a first end opening and a second end opening and a hollow space extending from the first end opening to the second end opening,
- a flange arranged around the outer surface of the tube,
- a plunger arranged partly inside the tube through the second end opening and being movable back-and-forth along the length axis of the tube,
**characterised in that** the flange is stationary arranged to a first distance from the first end opening of the tube.

2. The inserter according to claim 1, **characterised in that** the first distance is 4.5 - 6.5 cm, preferably 5 - 6 cm.

3. The inserter according to claim 1 or 2, **characterised in that** the tube is malleable and it is made of plastic.

4. The inserter according to claim 1, 2 or 3, **characterised in that** the flange is arranged to a second distance from the second end opening of the tube, the second distance being 20 - 30 cm.

5. The inserter according to any of the claims 1 - 4, **characterised in that** the width of the flange in its diametrical direction is 2 - 4 mm, and the length of the flange in axial direction of the tube is 2 - 5 mm.

6. A kit comprising
- an inserter according to any of the claims 1 to 5, and
- an intrauterine system comprising
- a T-shaped frame consisting of an elongate member having at its first end a transverse member comprising a first wing and a second wing, the first ends of said wings being attached to said elongate member, and the first and second wings being symmetrically bent towards a second end of said elongate member so that an angle α is formed between said elongate member and said first or second wing; and the second ends of the wings being symmetrically bent towards the elongate member, and
- a therapeutic system.

7. A kit according to claim 6, **characterised in that** the angle α formed between the elongate member and the first or second wings is typically 10°-60°, preferably 20° - 50°, more preferably 30° - 50°.

8. A kit according to claim 6 or 7, **characterised in that** the second ends of the IUS wings are symmetrically bent towards the elongate member by an angle β that is usually 40°-140°, preferably 60°-130°, more preferably 90°-120°.

9. A method for inserting an IUS into uterine cavity comprising following steps:
- loading an IUS to the IUS inserter tube through a first end opening of the tube,
- inserting an IUS inserter loaded with the IUS into the uterine cavity through vagina and through the cervical canal,
- releasing the IUS into the uterine cavity by using the inserter,
**characterised in**
- using an inserter comprising a flange, which is stationary arranged to a first distance from the first end opening of the tube without any measurement of the depth of the uterine cavity before or during the insertion procedure.
